# EUROPÄISCHE PATENTSCHRIFT

(11) **EP 1 482 881 B1**
(45) Veröffentlichungstag und Bekanntmachung des Hinweises auf die Patenterteilung: **20.07.2005**
(21) Anmeldenummer: 03708116.3
(22) Anmeldetag: 20.02.2003
(51) Int. Cl.: A61F 5/01

(54) **ORTHESE MIT EINSTELLBAREM BEWEGUNGSBEREICH**
ORTHESIS COMPRISING AN ADJUSTABLE RANGE OF MOVEMENT
ORTHESE A PLAGE DE DEPLACEMENT REGLABLE

(30) Priorität: 22.02.2002 DE 10207702
(43) Veröffentlichungstag der Anmeldung: 08.12.2004
(73) Patentinhaber: Albrecht GmbH, 83115 Neubeuern (DE)
(72) Erfinder: OPAHLE, Hans-Georg, 83024 Rosenheim (DE); ALBRECHT, Erich, 83115 Neubeuern (DE)
(74) Vertreter: Bauer, Friedrich
(86) Internationale Anmeldenummer: PCT/EP2003/001752
(87) Internationale Veröffentlichungsnummer: WO 2003/070129

(56) Entgegenhaltungen:
- WO-A-01/58392
- DE-A- 19 904 554
- US-B1- 6 203 511

## Beschreibung

Die Erfindung betrifft eine Orthese, beispielsweise eine Ellbogenorthese, insbesondere zur Reduktion von Streck- und/oder Beugedefiziten, gemäß dem Oberbegriff des Anspruches 1.

Insbesondere Gelenkkapseln und/oder Bindegewebe weisen beispielsweise nach Bänderoperationen, Unfällen, Entzündungen etc. häufig ein Streck- und/oder Beugedefizit auf. Dies bedeutet, dass ein distales Körperglied, beispielsweise ein Unterarm, nicht mehr vollständig in seine normale Extensions- oder Flexionslage bezüglich eines proximalen Körperglieds, beispielsweise eines Oberarms, gebracht werden kann.

Orthesen dienen bekannterweise dazu, derartige Gelenke insbesondere während der Bewegung zu führen. Weiterhin soll die Orthese die Bewegung des Gelenkes in Extensions- und/oder Flexionsrichtung derart begrenzen, dass Verletzungen aufgrund übermäßiger Beanspruchung ausgeschlossen werden.

Weiterhin werden zum Dehnen der Verkürzungen und Schrumpfungen im Bereich des Gelenkes auch sogenannte Quengelvorrichtungen verwendet, d.h. Orthesen, die unter Federvorspannung stehen und die Bewegung des Körpergliedes bis zur gewünschten Extensions- bzw. Flexionsgrenze unterstützen.

Bei Orthesen müssen der Extensions- und Flexionsanschlag innerhalb weiter Grenzen einstellbar sein, um den individuellen Bedürfnissen des Patienten gerecht zu werden. Weiterhin ist eine einfache und schnelle Verstellmöglichkeit der Schwenkbereichsgrenzen wünschenswert, um bei zunehmender Mobilisierung des Gelenkes die Schwenkbereichsgrenzen entsprechend einfach und schnell nachstellen zu können.

Bekannte, zum Quengeln verwendete Orthesen weisen zur Begrenzung des Schwenkbereichs Anschlagstifte auf, die in verschiedene, um die Schwenkachse herum angeordnete Bohrungen eingesteckt werden können. Nachteilig ist hierbei jedoch, dass das Verstellen des Extensions- und Flexionsanschlags nur in groben Schritten, beispielsweise in 15°-Schritten, möglich ist und die Schwenkbereichsgrenzen daher häufig nicht genau genug eingestellt werden können.

Aus der US 5,873,847 ist eine Orthese bekannt, bei welcher keine einsteckbaren Anschlagstifte, sondern zwei hintereinander angeordnete Anschlageinstellscheiben zur Einstellung der Schwenkbereichsgrenzen in Extensions- und Flexionsrichtung verwendet werden. Zum Arretieren dieser Anschlageinstellscheiben ist eine Feststelleinrichtung in der Form von zwei Druckplatten vorgesehen, die mittels einer Schraube zusammengespannt werden können und dadurch die dazwischen liegenden Anschlageinstellscheiben einklemmen. Nachteilig ist hierbei jedoch, dass zum Verstellen der Schwenkbereichsgrenzen ein Werkzeug erforderlich ist, um die Feststelleinrichtung lösen und wieder zusammenspannen zu können. Ein derartiges Werkzeug ist häufig gerade dann nicht zur Hand, wenn es benötigt wird. Weiterhin ist problematisch, dass die Anschlageinstellscheiben relativ fest zusammengespannt werden müssen, um ein unerwünschtes Drehen der Anschläge auszuschließen. Dies erfordert einen gewissen Kraftaufwand beim Zuspannen und Lösen der Feststelleinrichtung. Wird die Feststellschraube zu wenig festgedreht, kann es zu einer unerwünschten Verdrehung der Anschlageinstellscheiben und damit zu einer Überdehnung der Muskeln, Bänder oder Sehnen kommen.

Eine weitere Orthese gemäß dem Oberbegriff von Anspruch 1 ist aus der Patentschrift US 6,203 511 bekannt.

Der Erfindung liegt die Aufgabe zugrunde, eine Orthese der eingangs genannten Art zu schaffen, mit der der Flexions- und/oder Extensionsanschlag auf einfache, schnelle, genaue und sichere Weise einstellbar und arretierbar ist.

Diese Aufgabe wird erfindungsgemäß durch die Merkmale des Anspruchs 1 gelöst. Vorteilhafte Ausführungsformen der Erfindung sind in den weiteren Ansprüchen beschrieben.

Bei der erfindungsgemäßen Orthese weist die Feststelleinrichtung eine in Richtung der Schwenkachse verschiebbare, relativ zur ersten Schiene drehfest angeordnete Verriegelungsscheibe auf. Diese Verriegelungsscheibe ist durch Verschieben zwischen einer die Anschlageinstellscheibe radial übergreifenden Arretierstellung, in der die Verriegelungsscheibe mit der Anschlageinstellscheibe formschlüssig verriegelnd in Eingriff ist, und einer Freigabestellung bewegbar, in welcher die Verriegelungsscheibe außer Eingriff mit der Anschlageinstellscheibe ist.

Bei der erfindungsgemäßen Orthese wird somit die Verriegelung der mindestens einen Anschlageinstellscheibe mittels einer axial verschiebbaren Verriegelungsscheibe vorgenommen, die in der Eingriffsstellung mit der Anschlageinstellscheibe formschlüssig in Eingriff ist und in der Freigabestellung mit der Anschlageinstellscheibe nicht mehr in Kontakt ist. Hierbei bedeutet axial verschiebbar, dass die Verriegelungsscheibe in Richtung ihrer eigenen Drehachse und damit in Richtung der Schwenkachse des Schienengelenkes verschiebbar ist.

Dadurch, dass die drehfest angeordnete Verriegelungsscheibe nicht mehr kraftschlüssig, sondern formschlüssig mit der Anschlageinstellscheibe in Eingriff tritt, ist ein unerwünschtes Verdrehen der Anschlageinstellscheibe ausgeschlossen. Die Formschlussverbindung wird dabei zweckmäßigerweise dadurch geschaffen, dass die Anschlageinstellscheibe eine Außenverzahnung und die Verriegelungsscheibe eine Innenverzahnung aufweist, die mit der Außenverzahnung der Anschlageinstellscheibe in und außer Kämmeingriff bringbar ist.

Die Verschiebung der Verriegelungsscheibe in Axialrichtung erfolgt zweckmäßigerweise mittels eines Drehteils, beispielsweise eines drehbaren Deckelteils, das ein Innengewinde aufweist und mit einem Außengewinde der Verriegelungsscheibe in Eingriff ist. Wird das Drehteil bewegt, bewegt sich die Verriegelungsscheibe spindelartig in Axialrichtung und kann damit in und außer Eingriff mit der Anschlageinstellscheibe gebracht werden. Befindet sich die Verriegelungsscheibe außer Eingriff mit der Anschlageinstellscheibe, kann die Anschlageinstellscheibe in die gewünschte Drehposition gebracht werden, bis der zugeordnete Schwenkbereichsanschlag in der gewünschten Winkelstellung ist. Weiterhin wird durch entgegengesetztes Drehen des Drehteils die Verriegelungsscheibe wieder in Eingriff mit der Anschlageinstellscheibe gebracht und diese damit arretiert.

Die Einstellung und Arretierung des Extensions- und/oder Flexionsanschlags ist damit auf sehr einfache, schnelle, genaue, sichere Weise und ohne Werkzeug möglich.

Die Erfindung wird nachfolgend anhand der Zeichnungen beispielhaft näher erläutert. Es zeigen:
- Figur 1:: eine perspektivische Darstellung der erfindungsgemäßen Orthese,
- Figur 2:: eine Explosionsdarstellung der Einzelteile der Orthese von Figur 1,
- Figur 3:: einen Längsschnitt durch das Schienengelenk der Orthese von Figur 1,
- Figuren 4A und 4B :: eine Ansicht schräg von oben bzw. unten eines Gehäuses,
- Figur 5:: ein Längsschnitt durch ein Drehteil,
- Figur 6:: eine Draufsicht auf eine Abdeckplatte,
- Figuren 7A und 7B :: eine Draufsicht bzw. Seitenansicht einer Verriegelungsscheibe,
- Figur 8:: eine perspektivische Darstellung einer Anschlageinstellscheibe,
- Figur 9:: einen Längsschnitt durch das Gehäuse mit eingesetztem Drehblock eines Totpunktverstellmechanismus,
- Figur 10:: eine Darstellung gemäß Figur 9, wobei zusätzlich ein Arretierstift mit Druckfeder und ein Exzenterteil eingesetzt sind,
- Figur 11:: eine perspektivische Darstellung des Drehblocks,
- Figuren 12A bis 12C :: einen Längsschnitt durch das Gehäuse mit zwei Anschlageinstellscheiben und der Feststelleinrichtung zum Arretieren der Anschlageinstellscheiben, wobei sich die Verriegelungsscheibe in der Verriegelungsposition bzw. in zwei unterschiedlichen Freigabepositionen befindet, und
- Figur 13:: eine perspektivische Darstellung eines Abschnitts der zweiten Schiene mit montiertem Anschlagelement.

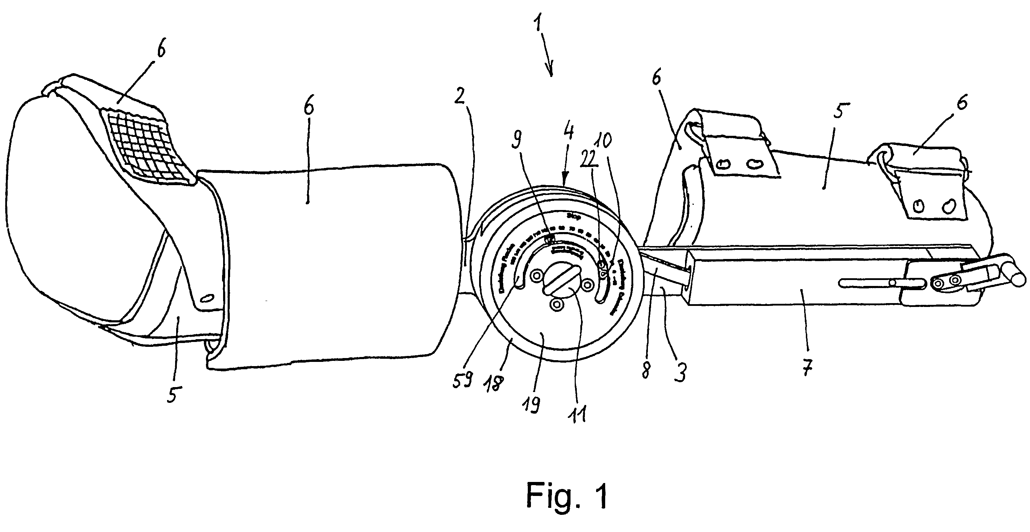

Aus Figur 1 ist eine Orthese 1 zur Reduzierung von Streck- und/oder Beugedefiziten von Ellbogengelenken mit einer ersten, am Oberarm zu befestigenden Schiene 2 und einer zweiten, am Unterarm zu befestigenden Schiene 3 ersichtlich, die mittels eines Schienengelenkes 4 gelenkig mit der ersten Schiene 2 verbunden ist. Die Befestigung am Ober- bzw. Unterarm erfolgt in bekannter Weise über Halbschalen 5, die an der ersten Schiene 2 bzw. zweiten Schiene 3 angebracht sind, und über Bänder oder Riemen 6, die um den Ober- bzw. Unterarm geschlungen werden.

Die dargestellte Orthese ist dazu ausgelegt, nur einseitig an das betreffende Körpergelenk angelegt zu werden. Es ist jedoch auch denkbar, für ein Körpergelenk zwei derartige Orthesen vorzusehen, die dann auf verschiedenen Seiten des Gelenkes angebracht werden.

Weiterhin weist die dargestellte Orthese einen Federkraftmechanismus mit einem Federgehäuse 7, in dem eine in Figur 1 nicht sichtbare Druckfeder angeordnet ist, sowie einer Druckstange 8 auf. Über die Druckstange 8 wird die Kraft der Druckfeder derart von der ersten Schiene 2 auf die zweite Schiene 3 übertragen, dass eine Schwenkkraft zwischen den beiden Schienen 2, 3 sowohl in Extensions- als auch in Flexionsrichtung erzeugt wird. Es handelt sich somit um eine zweiseitig wirkende Quengelvorrichtung. Da ein derartiger Federkraftmechanismus bereits bekannt ist, wird dieser nur soweit erläutert, als dies zum Verständnis der Erfindung erforderlich ist.

Die dargestellte Orthese ermöglicht auf einfache und genaue Weise sowie ohne Werkzeug ein Verstellen der Schwenkbereichsgrenzen in Extensions- und Flexionsrichtung mittels zweier Einstellstifte 9, 10, sowie das einfache Einstellen eines Totpunktes, ab dem der Federkraftmechanismus in Flexions- bzw. Extensionsrichtung wirkt, durch Betätigen eines Drehknopfs 11.

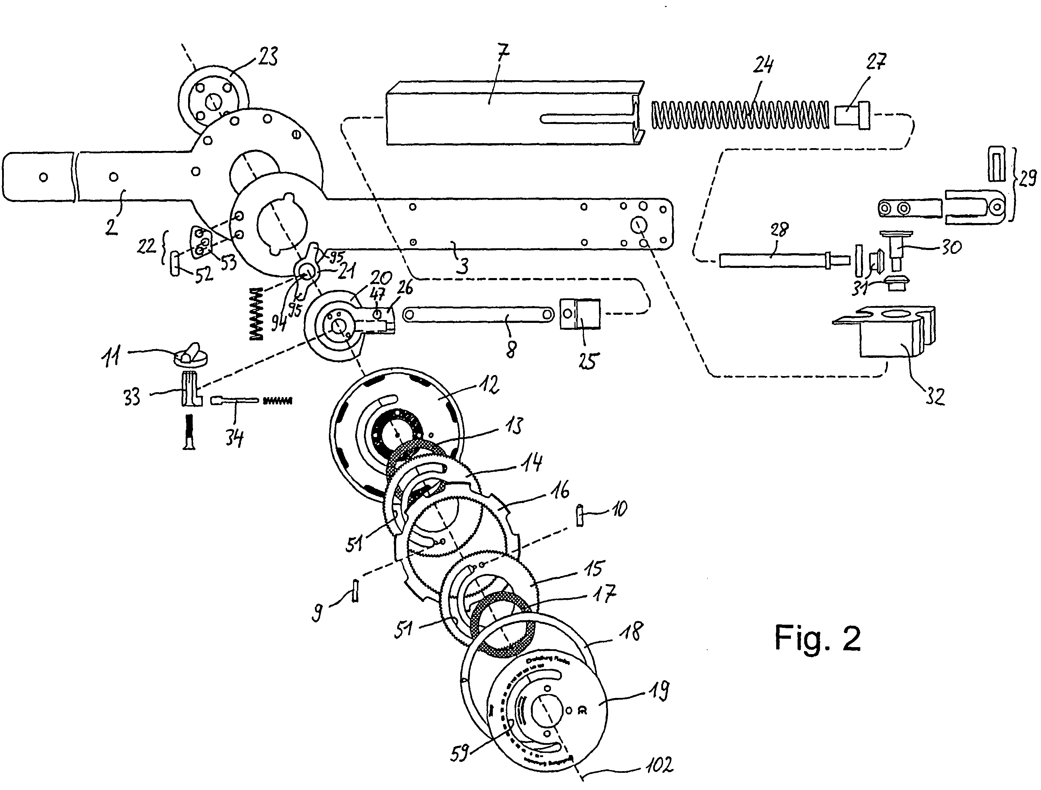

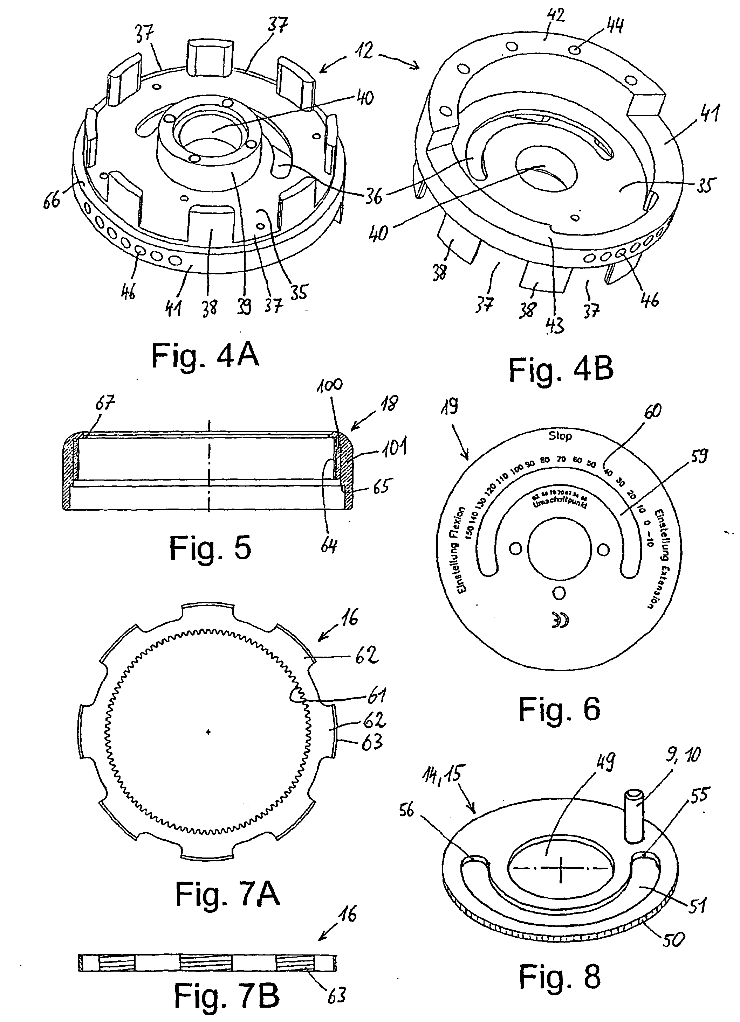

Die einzelnen Komponenten dieser Orthese werden im Folgenden anhand von Figur 2 näher erläutert. Die Schwenkachse ist dort mit 102 bezeichnet. Kernstück des Schienengelenkes 4, das die beiden Schienen 2, 3 miteinander verbindet, ist ein (in den Figuren 4A und 4B näher dargestelltes) Gehäuse 12, das in einem randseitigen Bereich mit der ersten Schiene 2 fest verschraubt ist. Auf der in Figur 2 vorderen Seite des Gehäuses 12 ist ein Elastomerring 13, eine erste Anschlageinstellscheibe 14 zum Einstellen des Extensionsanschlags, eine zweite Anschlageinstellscheibe 15 zur Einstellung des Flexionsanschlags, eine Verriegelungsscheibe 16, welche die Anschlageinstellscheiben 14, 15 umgibt, ein Elastomerring 17, ein (in Figur 5 näher dargestelltes) Drehteil 18 sowie eine Abdeckplatte 19 angeordnet.

Auf der in Figur 2 hinteren Seite des Gehäuses 12 ist ein zentraler Drehblock 20 zur Verstellung des Totpunktes des Federkraftmechanismus, ein im Drehblock 20 axial verschiebbar angeordneter Riegel 21, die zweite Schiene 3, auf der eine Anschlageinrichtung 22 festgeschraubt ist, die erste Schiene 2 sowie eine Halteplatte 23 angeordnet.

An der zweiten Schiene 3 ist das Federgehäuse 7 festgeschraubt, in der sich die Druckfeder 24 befindet. Die Druckfeder 24 liegt mit ihrem vorderen Ende an einem Druckzylinder 25 an, der gelenkig mit der Druckstange 8 verbunden ist. Am gegenüberliegenden Ende ist die Druckstange 8 gelenkig mit einem radial vorragenden Arm 26 des zentralen Drehblocks 20 verbunden. Über die Druckstange 8 wird somit fortwährend die Schubkraft der Druckfeder 24 auf den Arm 26 übertragen.

Am hinteren Ende ist die Druckfeder 8 an einem Federspannkolben 27 abgestützt, der als Spindelmutter mit Innengewinde ausgebildet ist. In diesem Federspannkolben 27 ist eine Federspannwelle 28 eingeschraubt, die eine Spindel darstellt. Diese Federspannwelle 28 kann über eine Klappkurbel 29, eine Kurbelwelle 30 und Getrieberäder 31 in Umdrehung versetzt werden, wodurch sich der Federspannkolben 27 in Axialrichtung bewegt, so dass die Vorspannkraft der Druckfeder 24 eingestellt werden kann. Die Getrieberäder 31 sind in einem Getriebegehäuse 32 gelagert.

Weiterhin ist in Figur 2 noch in Einzeldarstellung der Drehknopf 11 gezeigt, mit dem ein Exzenterteil 33 gedreht werden kann. Das Exzenterteil 33 dient, wie später noch näher erläutert wird, zum Verriegeln bzw. Freigeben eines radial angeordneten Arretierstifts 34, der im Arm 26 des Drehblocks 20 längsverschiebbar gelagert ist.

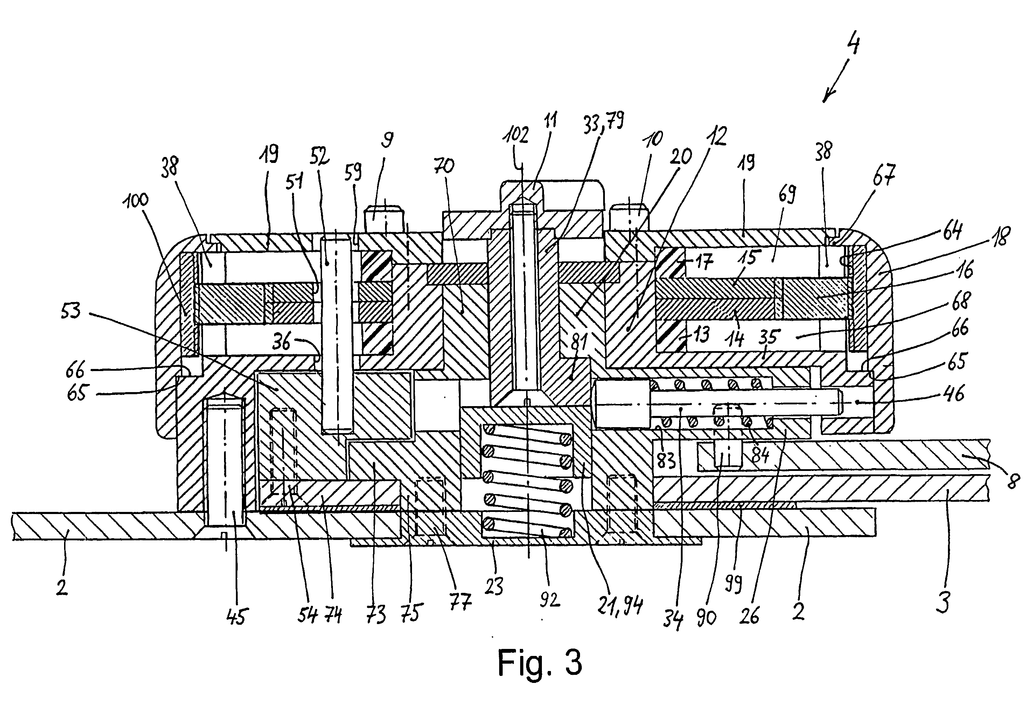

Im Folgenden werden nun die Einzelteile und deren Funktionsweise anhand der Figuren 3 bis 13 näher erläutert.

Das Gehäuse 12 ist in Figur 4A von der Vorderseite und in Figur 4B von der Rückseite her dargestellt. Das Gehäuse 12 weist eine ebene Mittelwand 35 auf, die durch einen kreisbogenförmigen Längsschlitz 36 durchbrochen ist. Der Längsschlitz 36 erstreckt sich über etwa 180°. Von der Mittelwand 35 erstreckt sich senkrecht eine durch regelmäßig über den Umfang verteilte Schlitze 37 unterbrochene Umfangswand 38 nach oben, die damit einzelne Finger bildet. Die Breite der Schlitze 37 entspricht in etwa der Breite der Finger. Weiterhin steht über die Mittelwand 35 ein zentraler Hülsenabschnitt 39 vor, der als Drehlager für die in Figur 8 dargestellten Anschlageinstellscheiben 14, 15 dient. Mittelwand 35 und Hülsenabschnitt 39 weisen eine durchgehende zentrale Bohrung 40 auf.

Über die Rückseite der Mittelwand 35 steht, wie aus Figur 4B ersichtlich, eine Umfangswand 41 vor. Die Höhe der Umfangswand 41 ist in einem ersten Umfangsabschnitt 42 höher als in einem zweiten Umfangsabschnitt 43. Im ersten Umfangsabschnitt 42 befinden sich mehrere axial ausgerichtete Gewindebohrungen 44, um die erste Schiene 2, die auf dem ersten Umfangsabschnitt 42 aufliegt, mittels Schrauben 45 (Figur 3) festschrauben zu können. Die Höhe des zweiten Umfangsabschnitts 43 ist über eine Umfangslänge von etwa 200° gegenüber dem ersten Umfangsabschnitt 42 reduziert, um einen entsprechenden Freiraum für die erste Schiene 2 und die Druckstange 8 zu schaffen, die sich radial über den zweiten Umfangsabschnitt 43 hinaus erstrecken. Im zweiten Umfangsabschnitt 43 sind weiterhin eine Mehrzahl von in Umfangsrichtung regelmäßig beabstandeten radialen Arretierbohrungen 46 vorgesehen, in die der Arretierstift 34 des zentralen Drehblocks 20 eingeführt werden kann, wodurch die Drehposition des Drehblocks 20 relativ zum Gehäuse 12 arretiert wird. Die Drehposition des Drehblocks 20 und damit diejenige des Anlenkpunktes 47 (Figur 2) der Druckstange 8 am Arm 26 kann somit dadurch eingestellt werden, dass der Drehblock 20 bei zurückgezogenem Arretierstift 34 zunächst bis zur gewünschten Drehposition gedreht wird, worauf der Arretierstift 34 in der gewünschten Arretierbohrung 46 eingerastet wird. Dieser Vorgang wird nachfolgend noch näher beschrieben.

Wie aus Figur 3 ersichtlich, sitzt auf der ebenen Mittelwand 35 des Gehäuses 12 der erste Elastomerring 13, der den zentralen Hülsenabschnitt 39 eng umgibt. Auf dem ersten Elastomerring 13 sitzt die erste Anschlageinstellscheibe 14 und darauf die zweite Anschlageinstellscheibe 15 sowie der Elastomerring 17.

Diese Anschlageinstellscheiben 14, 15 sind weitgehend identisch ausgebildet, so dass in Figur 8 nur eine dieser Anschlageinstellscheiben 14, 15 dargestellt ist. Die Anschlageinstellscheiben 14, 15 sind kreisförmig und weisen eine mittige Durchgangsöffnung 49 auf, so dass sie mit geringem Spiel auf den zentralen Hülsenabschnitt 39 des Gehäuses 12 aufgesteckt werden können. Der zentrale Hülsenabschnitt 39 bildet somit ein Drehlager für die Anschlageinstellscheiben 14, 15. Weiterhin können sich die Anschlageinstellscheiben 14, 15 auch noch geringfügig axial bewegen, soweit es die Elastomerringe 13, 17 erlauben, zwischen denen sie eingespannt sind und die aus relativ weichem Material bestehen.

An ihrem Außenumfang weisen die Anschlageinstellscheiben 14, 15 eine relativ feine Außenverzahnung 50 auf, die in Figur 8 schematisch dargestellt ist. Zwischen dem Außenumfang und der Durchgangsöffnung 49 erstreckt sich ein durchgehender, kreisbogenförmiger Längsschlitz 51 über einen Winkelbereich von etwa 200°. Die beiden Anschlageinstellscheiben 14, 15 werden so montiert, dass die beiden Längsschlitze 51 zumindest zum überwiegenden Teil zur Deckung gebracht werden können und diese wiederum über dem Schlitz 36 des Gehäuses 12 liegen. Auf diese Weise kann ein in Figur 3 dargestellter Anschlagstift 52, der über ein Sockelteil 53 und Schrauben 54 fest mit der zweiten Schiene 3 verbunden ist, sich von unten her durch die Längsschlitze 36, 51 nach oben erstrecken und sich längs dieser bewegen. Da die Anschlageinstellscheiben 14, 15 über die Außenverzahnungen 50 drehfest relativ zum Gehäuse 12 und damit zur ersten Schiene 2 arretiert sind, kann sich der Anschlagstift 52 soweit innerhalb der Längsschlitze 36, 51 bewegen, bis er am Ende der Längsschlitze 51 angelangt ist und entweder am Anschlag 55 oder am gegenüberliegenden Anschlag 56 anschlägt. Es ist somit erkennbar, dass die Position der Schwenkbereichsgrenzen von der Position der Anschläge 55, 56 relativ zum Gehäuse 12 und damit zur ersten Schiene 2 abhängt und dass durch unabhängiges Drehen der Anschlageinstellscheiben 14, 15 relativ zum Gehäuse 12 die Schwenkbereichsgrenzen in Flexions- und Extensionsrichtung unabhängig voneinander eingestellt werden können.

Um nach einer nachfolgend noch näher beschriebenen Entriegelung der Anschlageinstellscheiben 14, 15 diese in die gewünschte Drehposition drehen zu können, weist jede Anschlageinstellscheibe 14, 15, wie auch aus Figur 3 ersichtlich, einen Einstellstift 9, 10 auf, der nach oben durch einen kreisbogenförmigen Längsschlitz 59 der Abdeckplatte 19 (Figur 6) hindurchragt und über die Abdeckplatte 19 nach oben etwas vorsteht, so dass die Einstellstifte 9, 10 mit den Fingern bewegt werden können. Entlang dieses Längsschlitzes 59 ist eine entsprechende Grad-einteilung 60 auf der Abdeckplatte 19 aufgebracht, um die Position der Anschläge 55, 56 und damit die Schwenkbereichsgrenzen in Flexions- und Extensionsrichtung ablesen zu können.

Die Verriegelung der Anschlageinstellscheiben 14, 15 in der gewünschten Drehposition erfolgt über die Verriegelungsscheibe 16, die in den Figuren 7A und 7B näher dargestellt ist. Die Verriegelungsscheibe 16 ist ringförmig ausgebildet und weist einen lichten Innendurchmesser auf, der dem Außendurchmesser der Anschlageinstellscheiben 14, 15 entspricht. An der inneren Umfangswand trägt die Verriegelungsscheibe 16 eine Innenverzahnung 61, die mit der Außenverzahnung 50 der Anschlageinstellscheiben 14, 15 in Eingriff gebracht werden kann. Weiterhin weist die Verriegelungsscheibe 16 regelmäßig über ihren Außenumfang verteilte Radialvorsprünge 62 auf, deren Länge (in Umfangsrichtung) der Breite der Schlitze 37 in der Umfangswand 38 des Gehäuses 12 entsprechen. Ist die Verriegelungsscheibe 16 im Gehäuse 12 eingesetzt, erstrecken sich die Radialvorsprünge 62 durch die Schlitze 37 des Gehäuses 12 hindurch, so dass die Verriegelungsscheibe drehfest, jedoch axial verschiebbar, im Gehäuse 12 aufgenommen ist. Der Außendurchmesser der Radialvorsprünge 62 ist weiterhin etwas größer als der Außendurchmesser der Umfangswand 38 des Gehäuses 12, so dass die Radialvorsprünge 62 geringfügig radial nach außen über die Umfangswand 38 hinausstehen.

Die Verriegelungsscheibe 16 kann mittels des Drehteils 18 axial relativ zum Gehäuse 12 verschoben werden. Hierzu weisen die Radialvorsprünge 62 ein äußeres Gewinde 63 auf, das mit einem Innengewinde 64 (Figur 5) des Drehteils 18 in Eingriff ist. Das im Wesentlichen hülsenförmig ausgebildete Drehteil 18 bildet den radialen Außenmantel des Schienengelenkes 4, das den oberen Teil des Gehäuses 12 einschließlich der Umfangswand 41, in dem sich die Arretierungsbohrungen 46 befinden, überdeckt. Das Drehteil 18 ist axial unverschiebbar, jedoch drehbar am Gehäuse 12 festgelegt. Hierzu weist das Drehteil 18 in seinem unteren Drittel eine radial nach innen vorspringende Schulter 65 auf, die auf einem randseitigen Absatz 66 des Gehäuses 12 aufliegt (Figur 3) und das Drehteil 18 axial nach unten festgelegt. Eine Axialverschiebung des Drehteils 18 nach oben wird durch eine radial nach innen vorspringende Schulter 67 verhindert, die sich am oberen Ende des Drehteils 18 befindet und vom Rand der Abdeckplatte 19 übergriffen wird.

Wie aus Figur 3 weiter ersichtlich, ist zwischen der ebenen Mittelwand 35 und der ersten Anschlageinstellscheibe 14 aufgrund des dazwischen liegenden Elastomerrings 13 ein axialer Freiraum 68 vorhanden. In gleicher Weise befindet sich zwischen der zweiten Anschlageinstellscheibe 15 und der Abdeckplatte 19 aufgrund des dazwischen liegenden Elastomerrings 17 ein axialer Freiraum 69. Die Höhe der Freiräume 68, 69 ist etwas größer als die Dicke der Anschlageinstellscheibe 14 bzw. 15. Die Elastomerringe 13, 17 halten, da sie die gleiche Höhe haben, die Anschlageinstellscheiben 14, 15 - in Axialrichtung gesehen - in der Mitte zwischen der Mittelwand 35 und der Abdeckplatte 19.

Die Verriegelungsscheibe 16 bildet zusammen mit dem Drehteil 18 eine Feststelleinrichtung zum Lösen und Arretieren der Anschlageinstellscheiben 14, 15.

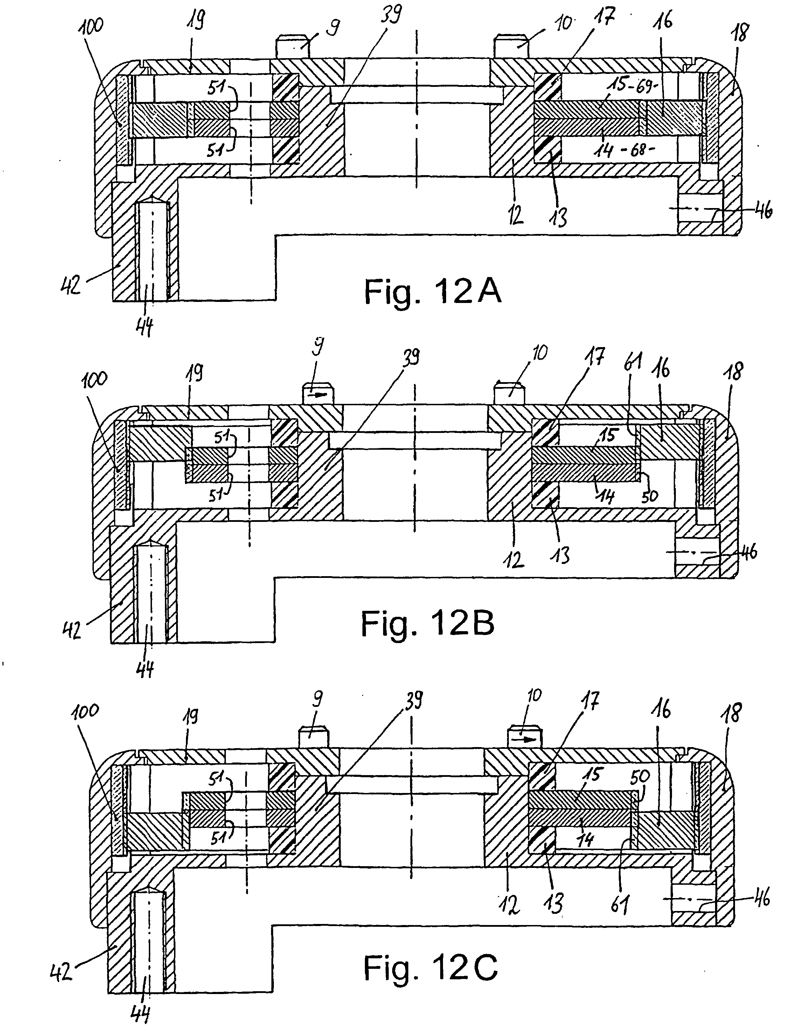

Die Verriegelungsscheibe 16, welche dieselbe Dicke hat wie die beiden Anschlageinstellscheiben 14, 15 zusammengenommen, kann durch Drehen des Drehteils 18 spindelartig nach oben oder unten verfahren werden, wie anhand der Figuren 12A bis 12C gezeigt. In Figur 12A befindet sich die Verriegelungscheibe 16 auf gleicher Höhe wie die beiden Anschlageinstellscheiben 14, 15. Beide Anschlageinstellscheiben 14, 15 sind daher mit der Innenverzahnung 61 der Verriegelungsscheibe 16 in Eingriff und durch diese drehfest arretiert. Wird das Drehteil 18 in einer bestimmten Richtung gedreht, kann die Verriegelungsscheibe 16 soweit nach oben verschoben werden, bis die untere Anschlageinstellscheibe 14 außer Eingriff mit der Verriegelungsscheibe 16 ist. Dieser Zustand ist in Figur 12B gezeigt. Die untere Anschlageinstellscheibe 14 kann daher von Hand mittels des Einstellstifts 9 in die gewünschte Drehposition gebracht werden, um beispielsweise den Extensionsanschlag einzustellen. Wird anschließend der Drehring 18 in die entgegengesetzte Richtung gedreht, kann die Verriegelungsscheibe 16 soweit nach unten bewegt werden, bis die obere Anschlageinstellscheibe 15 außer Eingriff mit der Verriegelungsscheibe 16 ist. Dieser Zustand ist in Figur 12C dargestellt. Die obere Anschlageinstellscheibe 15 kann nun mittels des Einstellstifts 10 in die gewünschte Stellung gedreht werden, um beispielsweise den Flexionsanschlag einzustellen. Sind beide Anschlageinstellscheiben 14, 15 richtig eingestellt, wird das Drehteil 18 wieder zurückgeschraubt, bis die Verriegelungsscheibe 16 beide Anschlageinstellscheiben 14, 15 verriegelt (Figur 12A).

Da es vorkommen kann, dass bei der Axialbewegung der Verriegelungsscheibe 16 von der in den Figuren 12B oder 12C gezeigten Position in die mittlere, in Figur 12A gezeigte Position die Innenverzahnung 61 der Verriegelungsscheibe 16 nicht gleich mit der Außenverzahnung 50 der Anschlageinstellscheiben 14, 15 kämmt, sondern Gewindezähne auf Gewindezähne stoßen, sind die Elastomerringe 13, 17 relativ weich ausgebildet und ermöglichen ein Nachgeben der Anschlageinstellscheiben 14, 15 in axialer Richtung. Hierdurch wird das Inneinandergreifen der Verzahnungen erleichtert.

In Figur 6 ist die Abdeckplatte 19 dargestellt. Wie ersichtlich, sind auf der Oberseite die Hinweise "Einstellung Flexion", "Stop" und "Einstellung Extension" angebracht. Diese Hinweise geben an, in welche Richtung das Drehteil 18 gedreht werden muss, um die Anschlageinstellscheibe für den Flexionsanschlag bzw. diejenige für den Extensionsanschlag frei zu geben. In der Position "Stop" sind beide Anschlageinstellscheiben 14, 15 verriegelt.

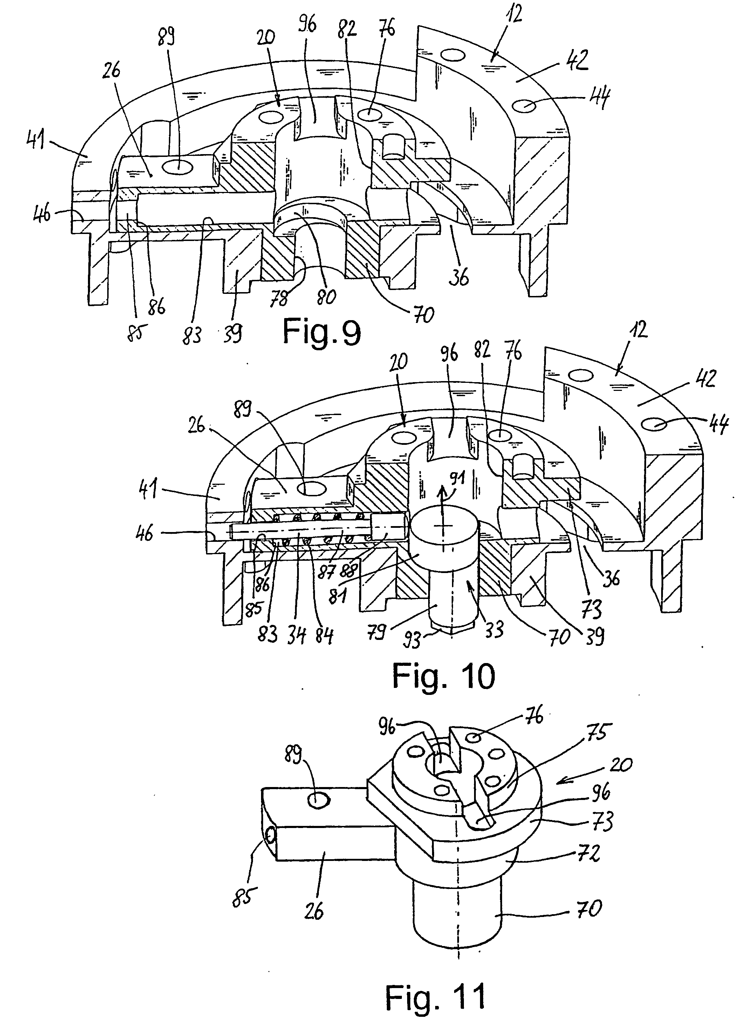

Anhand der Figuren 9 bis 11 wird nachfolgend der Totpunktverstellmechanismus näher beschrieben, mit dem der Totpunkt verstellt werden kann, ab dem die Wirkrichtung des Federkraftmechanismus von Flexion auf Extension bzw. umgekehrt umschaltet. Der zentrale Drehblock 20, der in Figur 11 in Einzeldarstellung gezeigt ist, weist hierzu einen zylindrischen Abschnitt 70 auf, der in die mittige Bohrung 40 des Gehäuses 12 eingesetzt und darin drehbar gelagert ist. An den zylindrischen Abschnitt 70 schließt sich ein zylindrischer Abschnitt 72 größeren Durchmessers an, an dem der Arm 26 derart angeformt ist, dass er sich radial vom Abschnitt 72 weg erstreckt. An den zylindrischen Abschnitt 72 schließt sich ein umlaufender Radialvorsprung 73 an. Dieser Radialvorsprung 73 dient als Anlagefläche für den kreisförmigen Endabschnitt 74 der zweiten Schiene 3. An den Radialvorsprung 73 schließt sich ein zylindrischer Abschnitt 75 an (Figur 11), der als Radiallager für die zweite Schiene 3 dient. Im zylindrischen Abschnitt 75 sind axiale Gewindebohrungen 76 vorgesehen, um die Halteplatte 23 mittels in Figur 3 angedeuteter Schrauben 77 festschrauben zu können.

Wie aus den Figuren 9 und 10 ersichtlich, befindet sich im zylindrischen Abschnitt 70 des Drehblocks 20 eine mittige axiale Bohrung 78, in dem ein Zylinderabschnitt 79 des Exzenterteils 33 drehbar gelagert ist. Die Bohrung 78 mündet in eine kreisförmige Tasche 80 größeren Durchmessers, die bezüglich der Bohrung 78 exzentrisch angeordnet ist. Die Tasche 80 dient zur Aufnahme eines exzentrischen Zylinderabschnitts 81 des Exzenterteils 33, so dass sich dieses nicht drehen kann, wenn der exzentrische Zylinderabschnitt 81 in der Tasche 80 liegt. Diese Position ist in Figur 10 dargestellt.

An die Tasche 80 schließt sich eine axiale, mittige Bohrung 82 größeren Durchmessers an. Der Durchmesser dieser Bohrung 82 ist derart bemessen, dass das Exzenterteil 33 beliebig gedreht werden kann, wenn es aus der in Figur 10 gezeigten Stellung in axialer Richtung, d.h. in Richtung des Pfeils 91, so weit nach oben verschoben worden ist, dass sich der exzentrische Zylinderabschnitt 81 oberhalb der Tasche 80 befindet.

Wie aus Figur 9 ersichtlich, erstreckt sich unmittelbar oberhalb der Tasche 80 eine radiale Bohrung 83 durch den gesamten Arm 26 hindurch, die zur Aufnahme des Arretierstifts 34 und einer Druckfeder 84 dient (Figur 10). Die Bohrung 83 weist in einem äußeren Endabschnitt 85 einen geringeren Durchmesser auf, so dass eine Schulter 86 gebildet wird, an der sich das äußere Ende der Druckfeder 84 abstützt. Der Arretierstift 34 weist einen Schaft 87 und einen Kopf 88 größeren Durchmessers auf, an dem das gegenüberliegende Ende der Druckfeder 84 abgestützt ist. Die Länge des Arretierstifts 34 ist weiterhin derart bemessen, dass sein äußeres Ende über den Arm 26 hinaus und in eine der Arretierbohrungen 46 hinein ragt, wenn das gegenüberliegende Ende des Kopfes 88 bündig mit der Wand der Bohrung 82 abschließt. Befindet sich in diesem Zustand, der in Figur 10 gezeigt ist, der exzentrische Zylinderabschnitt 81 des Exzenterteils 33 innerhalb der Tasche 80, kann sich der Arretierstift 34 nicht in die Bohrung 82 zurück bewegen, da er am exzentrischen Zylinderabschnitt 81 anstößt. Der Arretierstift 34 ist damit in einer eine Drehbewegung des Drehblocks 20 relativ zum Gehäus 12 verhindernden Weise verriegelt. Der Anlenkpunkt 47 (Figur 2) der durch eine axiale Bohrung 89 im Arm 26 (Figur 10) und durch einen in der Druckstange 8 befestigten und in die Bohrung 89 eingreifenden Gelenkstift 90 (Figur 3) gebildet wird, ist damit relativ zum Gehäuse 12 festgelegt.

Um den Anlenkpunkt 47 und damit den Totpunkt des Federkraftmechanismus zu verstellen, wird das Exzenterteil 33 in Richtung des Pfeils 91 (Figur 10) entgegen der Kraft einer Druckfeder 92 (Figur 3) mittels des Drehknopfs 11 verschoben, bis der exzentrische Zylinderabschnitt (81) außerhalb der Tasche 80 liegt. Das Exzenterteil 33 kann anschließend mittels des Drehknopfs 11, der über einen Vierkant 93 drehfest mit dem Exzenterteil 33 verbunden ist, um 180° gedreht werden, wodurch sich der Arretierstift 34 unter der Druckkraft der Druckfeder 84 in die Bohrung 82 zurück bewegt und das gegenüberliegende Ende des Arretierstifts 34 aus der Arretierbohrung 36 austreten kann. Der Drehblock 20 kann nun bis zu einer anderen gewünschten Arretierbohrung 46 weiter gedreht werden, worauf der Arretierstift 34 durch Zurückdrehen des Exzenterteils 33 wieder in die verriegelnde Eingriffsposition gebracht wird.

Die Drehung des Drehteils 20 in die gewünschte Position relativ zum Gehäuse 12 erfolgt bei entriegeltem Arretierstift 33 mittels der zweiten Schiene 3, die über den in den Figuren 2 und 3 dargestellten Riegel 21 drehfest mit dem Drehblock 20 verbunden werden kann. Der Riegel 21 weist ein napfförmiges Mittelteil 94 auf, von dem aus sich zwei Flügel 95 nach entgegengesetzten Seiten erstrecken.

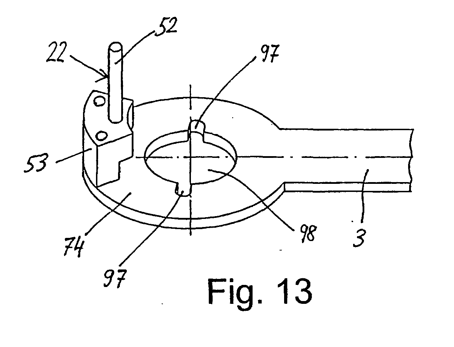

Im normalen Betrieb der Orthese, d.h. wenn sich in Figur 10 das Exzenterteil 33 in der dargestellten tiefsten Stellung befindet, ist das napfförmige Mittelteil 94 des Riegels 21 in der tiefstmöglichen Position innerhalb der Bohrung 82 angeordnet, wobei die Flügel 95 tief in Radialnuten 96 des zentralen Drehblocks 20 (Figur 11) liegen. In diesem Zustand stehen die Flügel 95 nach oben nicht über den Radialvorsprung 73 vor, so dass sich die zweite Schiene 3 um den zylindrischen Abschnitt 75 des Drehblocks 20 drehen kann. Wird jedoch zum Entriegeln des Arretierstifts 34 das Exzenterteil 33 nach oben geschoben (s. Pfeil 91 in Figur 10), wird hierdurch auch der Riegel 21 nach oben geschoben, wodurch die Flügel 95 axial in den Bereich des zylindrischen Abschnitts 75 und in seitliche Nuten 97 (Figur 13) hineingeschoben werden können, die sich von der zentralen Lagerbohrung 98 der zweiten Schiene 3 aus radial nach außen erstrecken. Hierdurch ist die zweite Schiene 3 drehfest mit dem Drehblock 20 gekoppelt, so dass dieser mittels der zweiten Schiene 3 in der gewünschten Weise verdreht werden kann, wenn der Arretierstift 34 entriegelt ist.

Das napfförmige Mittelteil 94 des Riegels 21 dient, wie aus Figur 3 ersichtlich, zum Abstützen der Druckfeder 92. Das gegenüberliegende Ende der Druckfeder 92 ist an der äußeren, mittigen Halteplatte 23 abgestützt. Wird das Exzenterteil 33 derart gedreht, dass der exzentrische Zylinderabschnitt 81 wieder in die Tasche 80 des Drehblocks 20 gelangen kann, drückt die Druckfeder 92 den Riegel 21 zusammen mit dem Exzenterteil 33 in die in Figur 3 dargestellte Position zurück. Dadurch gelangen die Flügel 95 des Riegels 21 außer Eingriff mit den seitlichen Nuten 97 der zweiten Schiene 3, so dass diese wieder innerhalb der eingestellten Schwenkbereichsgrenzen um den Drehblock 20 und damit relativ zur ersten Schiene 2 geschwenkt werden kann.

Um die Reibung zwischen dem kreisförmigen Endteil der ersten Schiene 2 und dem kreisförmigen Endteil der zweiten Schiene 3 zu minimieren, ist zwischen diesen Endteilen, wie aus Figur 3 ersichtlich, eine Zwischenscheibe 99 eingesetzt.

In dem dargestellten Ausführungsbeispiel ist das Innengewinde 64 des Drehteils 18 (Figur 5) aus fertigungstechnischen Gründen auf einem separaten Gewindering 100 angebracht, der mittels eines geeigneten Klebers mit einem Mantel 101 des Drehteils 18 verbunden ist. Alternativ hierzu ist es jedoch auch ohne weiteres möglich, das Innengewinde 64 direkt am Mantel 101 auszubilden.

Die Erfindung wurde anhand eines Beispiels einer Ellbogenorthese beschrieben, kann jedoch auch für andere Orthesen, beispielsweise Knieorthesen, Verwendung finden.

## Patentansprüche

1. Orthese mit
- einer ersten Schiene (2), die an einem ersten Körperglied befestigbar ist,
- einer zweiten Schiene (3), die an einem zweiten Körperglied befestigbar ist,
- einem Schienengelenk (4) zur schwenkbaren Verbindung der ersten und zweiten Schiene (2, 3),
- mindestens einer um eine Schwenkachse (102) drehbare und in unterschiedlichen Drehpositionen arretierbaren Anschlageinstellscheibe (14, 15) zur Einstellung einer Schwenkbereichsgrenze, und
- einer Feststelleinrichtung zum Arretieren der mindestens einen Anschlageinstellscheibe (14, 15),
**dadurch gekennzeichnet, dass** die Feststelleinrichtung eine in Richtung der Schwenkachse (102) verschiebbare, relativ zur ersten Schiene (2) drehfest angeordnete Verriegelungsscheibe (16) aufweist, welche durch Verschieben zwischen einer die Anschlageinstellscheibe (14, 15) radial übergreifenden Arretierstellung, in der die Verriegelungsscheibe (16) mit der Anschlageinstellscheibe (14, 15) formschlüssig verriegelnd in Eingriff ist, und einer Freigabestellung bewegbar ist, in welcher die Verriegelungsscheibe (16) außer Eingriff mit der Anschlageinstellscheibe (14, 15) ist.

2. Orthese nach Anspruch 1, **dadurch gekennzeichnet, dass** die Anschlageinstellscheibe (14, 15) eine Außenverzahnung (50) und die Verriegelungsscheibe (16) eine Innenverzahnung (61) aufweisen, die mit der Außenverzahnung (50) der Anschlageinstellscheibe (14, 15) in und außer Kämmeingriff bringbar ist.

3. Orthese nach Anspruch 1 oder 2, **dadurch gekennzeichnet, dass** die Verriegelungsscheibe (16) ein Gewinde (63) aufweist und über das Gewinde (63) spindelartig bewegbar ist.

4. Orthese nach einem der vorhergehenden Ansprüche, **dadurch gekennzeichnet, dass** die Verriegelungsscheibe (16) an ihrer radialen Außenumfangsfläche ein Gewinde (63) aufweist, und dass die Feststelleinrichtung ein die Verriegelungsscheibe (16) radial umgebendes, axial festgelegtes Drehteil (18) mit einem Innengewinde (64) aufweist, das mit dem Gewinde (63) der Verriegelungsscheibe (16) in Eingriff ist und durch Drehen eine axiale Verschiebung der Verriegelungsscheibe (16) bewirkt.

5. Orthese nach einem der vorhergehenden Ansprüche, **dadurch gekennzeichnet, dass** zwei Anschlageinstellscheiben (14, 15) zur Einstellung der Schwenkbereichsgrenzen in Extensions- und Flexionsrichtung vorgesehen sind, die zueinander parallel und benachbart angeordnet und gleichzeitig durch die selbe Verriegelungsscheibe (16) arretierbar sind.

6. Orthese nach Anspruch 5, **dadurch gekennzeichnet, dass** die Feststelleinrichtung derart ausgebildet ist, dass durch Verschieben der Verriegelungsscheibe (16) in die eine Richtung die Extensions-Anschlageinstellscheibe (14) freigegeben wird und durch Verschieben der Verriegelungsscheibe (16) in die entgegengesetzte Richtung die Flexions-Anschlageinstellscheibe (15) freigegeben wird.

7. Orthese nach einem der vorhergehenden Ansprüche, **dadurch gekennzeichnet, dass** das Schienengelenk (4) ein mit der ersten Schiene (2) fest verbundenes Gehäuse (12) mit einer die Verriegelungsscheibe (16) teilweise umgebenden Umfangswand (38) aufweist, dass die Verriegelungsscheibe (16) Radialvorsprünge (62) aufweist, und dass in der Umfangswand (38) Schlitze (37) vorgesehen sind, durch welche die Radialvorsprünge (62) hindurchgeführt sind, um ein Drehen der Verriegelungsscheibe (16) zu verhindern.

8. Orthese nach Anspruch 7, **dadurch gekennzeichnet, dass** das Gehäuse (12) einen zentralen Hülsenabschnitt (39) aufweist, der als Drehlager für die mindestens eine Anschlageinstellscheibe (14, 15) ausgebildet ist, dass ein Federkraftmechanismus vorgesehen ist, um die zweite Schiene (3) gegenüber der ersten Schiene (2) sowohl in Extensionsrichtung als auch in Flexionsrichtung vorzuspannen, und dass innerhalb des Hülsenabschnitts (39) eine Totpunktverstelleinrichtung für den Federkraftmechanismus drehbar gelagert ist.

9. Orthese nach Anspruch 8, **dadurch gekennzeichnet, dass** der Totpunktverstellmechanismus einen Drehblock (20) umfasst, in dem ein Arretierstift (34) quer zur Schwenkachse (102) verschiebbar geführt ist, dass das Gehäuse (12) eine Mehrzahl von radialen Arretierbohrungen (46) aufweist, die in Umfangsrichtung des Gehäuses (12) beabstandet angeordnet sind, und dass innerhalb des Drehblocks (20) ein Exzenterteil (33) drehbar gelagert ist, um den Arretierstift (34) in Eingriff mit einer Arretierbohrung (46) zu halten oder das Entfernen des Arretierstifts (34) aus der Arretierbohrung (46) zu ermöglichen.

10. Orthese nach Anspruch 9, **dadurch gekennzeichnet, dass** der Drehblock (20) des Totpunktverstellmechanismus als Drehlager für die zweite Schiene (3) ausgebildet ist.

## Claims

1. Orthesis with
- a first bar (2) which can be fastened to a first body part,
- a second bar (3) which can be fastened to a second body part,
- a bar hinge (4) for pivotable connection of the first and second bars (2, 3),
- at least one click-stop dial (14, 15) which is rotatable about a pivot axis (102), can be blocked in different rotation positions and is used for adjusting a pivot range limit, and
- a fixing device for blocking the at least one click-stop dial (14, 15),
**characterized in that** the fixing device has a locking disc (16) which is displaceable in the direction of the pivot axis (102), is mounted in a rotationally fixed manner in relation to the first bar (2) and can be moved, by being displaced, between a blocking position, in which the locking disc (16) engages radially over the click-stop dial (14, 15) and is in locked form-fit engagement with said click-stop dial (14, 15), and a release position, in which the locking disc (16) is disengaged from the click-stop dial (14, 15).

2. Orthesis according to Claim 1, **characterized in that** the click-stop dial (14, 15) has an outer toothing (50), and the locking disc (16) has an inner toothing (61) which can be moved into and out of meshing engagement with the outer toothing (50) of the click-stop dial (14, 15).

3. Orthesis according to Claim 1 or 2, **characterized in that** the locking disc (16) has a thread (63) and can be moved in the manner of a spindle via the thread (63).

4. Orthesis according to one of the preceding claims, **characterized in that** the locking disc (16) has a thread (63) on its radial outer circumferential surface, and **in that** the fixing device has an axially fixed rotation part (18) which radially surrounds the locking disc (16) and has an internal thread (64), which rotation part engages with the thread (63) of the locking disc (16) and, when rotated, causes an axial displacement of the locking disc (16).

5. Orthesis according to one of the preceding claims, **characterized in that** two click-stop dials (14, 15) are provided for adjusting the pivot range limits in the extension direction and flexion direction, said click-stop dials (14, 15) being arranged parallel and next to one another and being able to be blocked simultaneously by the same locking disc (16).

6. Orthesis according to Claim 5, **characterized in that** the fixing device is designed in such a way that, by displacing the locking disc (16) in one direction, the extension click-stop dial (14) is released, and, by displacing the locking disc (16) in the opposite direction, the flexion click-stop dial (15) is released.

7. Orthesis according to one of the preceding claims, **characterized in that** the bar hinge (4) has a housing (12) fixedly connected to the first bar (2) and with a circumferential wall (38) partially surrounding the locking disc (16), **in that** the locking disc (16) has radial projections (62), and **in that** the peripheral wall (38) is provided with slits (37) through which the radial projections (62) are guided in order to prevent rotation of the locking disc (16).

8. Orthesis according to Claim 7, **characterized in that** the housing (12) has a central sleeve portion (39) designed as a rotation bearing for the at least one click-stop dial (14, 15), **in that** a spring force mechanism is provided in order to pretension the second bar (3) relative to the first bar (2) both in the extension direction and in the flexion direction, and **in that** a dead-point adjustment device for the spring force mechanism is mounted rotatably inside the sleeve portion (39).

9. Orthesis according to Claim 8, **characterized in that** the dead-point adjustment mechanism comprises a rotation block (20) in which a blocking pin (34) is displaceably guided transversely with respect to the pivot axis (102), **in that** the housing (12) has a plurality of radial blocking bores (46) which are spaced apart in the circumferential direction of the housing (12), and **in that** an eccentric part (33) is mounted rotatably inside the rotation block (20) in order to keep the blocking pin (34) in engagement with a blocking bore (46) or to permit removal of the blocking pin (34) from the blocking bore (46).

10. Orthesis according to Claim 9, **characterized in that** the rotation block (20) of the dead-point adjustment mechanism is designed as a rotation bearing for the second bar (3).

## Revendications

1. Orthèse, comprenant :
-- une première tringle (2) qui peut être fixée à un premier membre corporel,
-- une seconde tringle (3) qui peut être fixée à un second membre corporel,
-- une articulation de tringle (4) pour une liaison pivotante de la première tringle et de la seconde tringle (2, 3),
-- au moins une plaque de réglage de butée (14, 15), capable de tourner autour d'un axe de pivotement (102) et d'être arrêtée dans des positions de rotations différentes, pour régler une limite de plage de pivotement, et
-- un moyen d'immobilisation pour arrêter ladite au moins une plaque de réglage de butée (14, 15),
**caractérisée en ce que** le moyen d'immobilisation comprend une plaque de verrouillage (16), en translation en direction de l'axe de pivotement (102) et agencée solidairement en rotation par rapport à la première tringle (2), laquelle est mobile par translation entre une position d'arrêt qui coiffe radialement la plaque de réglage de butée (14, 15), dans laquelle la plaque de verrouillage (16) est en engagement de verrouillage par coopération de formes avec la plaque de réglage de butée (14, 15), et une position de libération, dans laquelle la plaque de verrouillage (16) n'est pas en engagement avec la plaque de réglage de butée (14, 15).

2. Orthèse selon la revendication 1, **caractérisée en ce que** la plaque de réglage de butée (14, 15) présente une denture extérieure (50) et la plaque de verrouillage (16) présente une denture intérieure (61), qui peut être amenée en engrènement avec la denture extérieure (50) de la plaque de réglage de butée (14, 15), et hors d'un tel engrènement.

3. Orthèse selon l'une ou l'autre des revendications 1 et 2, **caractérisée en ce que** la plaque de verrouillage (16) présente un pas de vis (63), et est susceptible d'être déplacée à la manière d'une broche au moyen du pas de vis (63).

4. Orthèse selon l'une des revendications précédentes, **caractérisée en ce que** la plaque de verrouillage (16) présente un pas de vis (63) sur sa surface périphérique extérieure radiale, et **en ce que** le moyen d'immobilisation comprend une pièce rotative (18) immobilisée axialement et entourant radialement la plaque de verrouillage (16), ladite pièce rotative (18) présentant un pas de vis intérieur (64) en engagement avec le pas de vis (63) de la plaque de verrouillage, et provoquant par rotation un déplacement axial de la plaque de verrouillage (16).

5. Orthèse selon l'une des revendications précédentes, **caractérisée en ce qu'**il est prévu deux plaques de réglage de butée (14, 15) pour le réglage des limites de la plage de pivotement dans la direction d'extension et dans la direction de flexion, lesdites plaques étant agencées parallèlement l'une à l'autre et au voisinage l'une de l'autre, et pouvant être arrêtées simultanément par la même plaque de verrouillage (16).

6. Orthèse selon la revendication 5, **caractérisée en ce que** le moyen d'immobilisation est réalisé de telle manière qu'en déplaçant la plaque de verrouillage (16) dans une direction, la plaque de réglage de butée en extension (14) est libérée, et en déplaçant la plaque de verrouillage (16) dans la direction opposée, la plaque de réglage de butée en flexion (15) est libérée.

7. Orthèse selon l'une des revendications précédentes, **caractérisée en ce que** l'articulation de tringle (4) comprend un boîtier (12) fermement relié au premier rail (2) et comportant une paroi périphérique (38) qui entoure partiellement le disque de verrouillage (16), **en ce que** le disque de verrouillage (16) comporte des saillies radiales (62), et **en ce qu'**il est prévu des fentes (37) dans la paroi périphérique (38), à travers lesquelles sont passées les saillies radiales (62) pour empêcher une rotation de la plaque de verrouillage (16).

8. Orthèse selon la revendication 7, **caractérisée en ce que** le boîtier (12) comprend un tronçon central en forme de douille (39), qui est réalisé comme palier de rotation pour ladite au moins une plaque de réglage de butée (14, 15), **en ce qu'**il est prévu un mécanisme de force à ressort pour précontraindre la seconde tringle (3) par rapport à la première tringle (2) aussi bien en direction d'extension qu'en direction de flexion, et **en ce qu'**à l'intérieur du tronçon en forme de douille (39) est monté en rotation un dispositif de déplacement à dépassement de point mort pour le mécanisme de force à ressort.

9. Orthèse selon la revendication 8, **caractérisée en ce que** le mécanisme de déplacement à dépassement de point mort comprend un bloc rotatif (20), dans lequel une tige d'arrêt (34) est guidée en déplacement transversalement à l'axe de pivotement (102), **en ce que** le boîtier (12) présente une pluralité de perçages d'arrêt radiaux (46), qui sont agencés à distance en direction périphérique du boîtier (12), et **en ce qu'**une partie à excentrique (33) est montée en rotation à l'intérieur du bloc rotatif (20), pour maintenir la tige d'arrêt (34) en engagement dans un perçage d'arrêt (46), ou pour permettre la sortie de la tige d'arrêt (34) hors du perçage d'arrêt (46).

10. Orthèse selon la revendication 9, **caractérisée en ce que** le bloc rotatif (20) du mécanisme de déplacement à dépassement de point mort est réalisé sous la forme de palier de rotation pour la seconde tringle (3).
